# EUROPEAN PATENT SPECIFICATION

(11) **EP 1 847 291 B1**
(45) Date of publication and mention of the grant of the patent: **15.12.2010**
(21) Application number: 07015405.9
(22) Date of filing: 04.11.1999
(51) Int. Cl.: A61N 1/05

(54) **Extractable implantable medical lead**
Extrahierbare und implantierbare medizinische Elektrode
Fil médical implantable et extractible

(30) Priority: 09.11.1998 US 188491; 09.11.1998 US 188858; 09.11.1998 US 188859
(43) Date of publication of application: 24.10.2007
(62) Divisional of application: 99971729.1
(73) Proprietor: Medtronic, Inc., Minneapolis, MN 55432-5604 (US)
(72) Inventor: Shoberg, Bret R., Hanover, MN 55341 (US); Padgett, Clare E., Minneapolis, MN 55419 (US); Laske, Timothy G., Shoreview, MN 55126 (US); Waldhauser, Steven L., Circle Pines, MN 55104 (US); Stewart, Mark T., Lino Lakes, MN 55014 (US); Taylor, Catherine E., Fridley, MN 55432 (US); Keeney, Kenneth W., Emily, MN 56447 (US); Marshall, Mark T., Forest Lake, MN 55025 (US)
(74) Representative: Hughes, Andrea Michelle

(56) References cited:
- EP-A- 0 539 148
- EP-A- 0 545 540
- US-A- 3 416 534
- US-A- 4 972 846

## Description

This invention relates generally to medical leads and more particularly to implantable cardiac leads.

In the context of implantable leads, and particularly in the context of implantable cardiac leads, there is often a need to remove a lead after it has been implanted in a patient's body for some period of time. In conjunction with lead removal, it is often necessary to apply traction to the lead, in order to pull it free from tissue adhering thereto. It has therefore been recognized for some time that a reinforcement of some type, extending along the lead body would be beneficial, in order to prevent breakage or partial disassembly of the lead during removal. For example, in U.S. Patent No. 5,231,996 issued to Bardy et al., a variety of reinforcement mechanisms are disclosed, including cords, filaments, braids, and the like.

Document EP-A-0 539 148 disloses the most relevant prior art.

More recently, in the context of implantable cardiac leads, the use of cabled or stranded conductors in place of the previously more commonly employed coiled conductors has become more popular. These cabled or stranded conductors, such as disclosed in U.S. Patent No. 5,584,873 issued to Shoberg et al., U.S. Patent No. 5,760,341 issued to Laske et al. and U.S. Patent No.5,246,014 issued to Williams et al. inherently provide an increased tensile strength lead, at least along the segment between the point at which the stranded or cabled conductor is coupled to an electrode and the point at which the conductor is coupled to an electrical connector at the proximal end of the lead. While this new conductor inherently provides a lead of enhanced tensile strength, in most transvenous cardiac pacing leads employing cabled or stranded conductors, the conductor which extends to the distal-most portion of the lead is still a coiled conductor in order to permit passage of a stylet. This distal-most portion of the lead, particularly in the context of leads employing tines or other passive fixation mechanisms, is the portion of the lead to be most likely to be firmly embedded in fibrous tissue. It is therefore desirable that this portion of the lead in particular should be capable of withstanding high tensile forces without breakage.
US-A-5,609,621 discloses a lead having proximal distal and intermediate sections and having an elongated defibrillation electrode extending along the intermediate section.
The lead disclosed in the present application is particularly designed to reduce problems associated with extraction after implant. In order to accomplish this goal, the lead is provided with three structural features, each directed particularly to providing a lead which is easier to extract and less likely to be damaged during the extraction process.
According to the invention, there is provided a medical electrical lead according to claim 1.

Preferably, the tip-ring assembly is adapted for use in conjunction with electrodes employing passive fixation mechanisms such as tines. Preferably, the tip-ring assembly includes a tine sleeve having a central lumen into which a proximal extending shank portion of the tip electrode is inserted, a tip-ring spacer component, adapted to be glued to the proximal end of the tine sleeve and a ring-coil spacer component, adapted to be glued to the tip-ring spacer component, and around which a ring electrode may be located. The ring-coil and tip-ring spacer components together define a circumferential groove dimensioned to receive and retain the ring electrode. The distal end of the tip-ring spacer is configured to overlap the proximal end of the electrode shank located within the tine sheath, so that a generally rigid assembly is provided extending from the distal or tip electrode through and including the ring electrode of the lead. The ring electrode is coupled to a stranded or cabled conductor which extends to the proximal end of the lead, which together with the components of the tip-ring assembly provide a first mechanism for transmission of tensile force applied to the proximal end of the lead all the way to the distal or tip electrode.

The description also relates to the provision of insulative coatings or tubings covering these strand and/or coiled conductors employed in the lead which have been treated to enhance their bonding performance, so that they may usefully be adhered to molded or extruded plastic components at either end of the lead, further providing for an additional mechanism of transmission of tensile force along the lead body. In this context, the conductor coupled to the tip electrode may be a coiled conductor surrounded by a heat shrink tube of polytetrafloroethelene (PTFE) which has been treated by etching or otherwise to enhance the ability to bond thereto. The distal end of the heat shrink tube may be bonded adhesively to one or more of the tine sleeve, the ring-coil spacer component and the tip-ring spacer component and to the connector assembly at the proximal end of the lead. The heat shrink PTFE tubing in conjunction with the associated coiled conductor and the adhesive bonds at the proximal and distal end of the lead provide a second mechanism for providing enhanced tensile strength extending along the entire length of the lead. The cabled conductor coupled to the ring electrode referred to above may correspondingly be provided with a plastic insulative coating, treated to improve adhesion. For example, the cabled conductor may be provided with a coating of ETFE, modified by plasma coating using silane gas to provide for increased bonding capabilities. The insulative coating on the cabled conductor may likewise be bonded to plastic components located at the proximal and distal ends of the lead, in turn allowing for distribution of tensile forces between the mechanical joints coupling the cabled conductor to the metal electrode and electrical connector components located at the distal and proximal ends of the leads respectively and adhesive bonds between the insulation and associated nearby plastic parts. The insulation may, for example be bonded to the molded parts associated with the tip-ring spacer and the connector assembly and/or to the extruded plastic tubing making up the lead body. By this mechanism, the ability of the cabled conductors to transmit tensile forces from the proximal end of the lead to the distal portion of the lead without damage to the lead is further enhanced. The improved bonding characteristics provided by surface treatment of the isulative coatings and/or tubes also assist in maintaining effective seals against fluid intrusion and migration within the lead body.

The lead of the present invention may also improve its extraction characteristics and is directed specifically to leads of the type employing elongated coil electrodes, for example as in implantable cardioversion and defibrillation leads. In some leads of this type, the coil is molded into the lead body, such as in U.S. Patent No. 4,161,952 issued to Kinney et al. However, a simpler alternative construction mechanism is to simply mount coil electrodes fabricated of single or multifilar coils around the exterior of an extruded tubular lead body. Such coil electrodes are disclosed in U.S. Patent No. 4,934,049 issued to Kiekhafer et al., U.S. Patent No. 5,115,818 issued to Holleman et al. and U.S. Patent No. 5,676,694 issued to Boser et al.

Experience has shown that discontinuities in lead diameter associated with the proximal and distal ends of such coil electrodes can complicate removal of the lead from its overlying fibrous sheath. This is true whether the removal is accomplished by attempting to remove the fibrous sheath prior to extraction of the lead or whether the lead is to be simply pulled through the fibrous sheath. According to this feature of the invention, tubing is provided overlying the extruded lead body intermediate the coil electrodes, if there is more than one such electrode and intermediate the proximal end of the most proximal coil electrode and the connector assembly located at the proximal end of the lead. In this fashion, a lead can be provided which is essentially isodiametric along the length of the lead body to the distal end of the distal-most coil electrode, which lead can be fabricated of extruded multi-lumen tubing and which does not require molding the coil electrode into the lead body. Preferably, if a ring electrode is located distal to the distal-most coil electrode, it too is configured to be essentially isodiametric to the electrode coil and to the lead body or other plastic component separating the distal end of the distal-most coiled conductor and the ring electrode. This particular construction mechanism is especially convenient in the context of a lead of the type employing an extruded multi-lumen tubular lead body, such as that described in the above cited patent issued to Shoberg et al. The provision of tubing overlying the extruded tubular lead body also provides for increased protection of the conductors therein without an over-all increase in lead diameter. This use of tubing which is of increased durability and/or greater insulative strength further enhances this benefit of the lead body structure.

Preferred embodiments will now be described, by way of example only with reference to the drawings.
Figure 1 is a plan view of a lead according to the present invention, provided with two coil electrodes.
Figure 2 is a sectional view through the distal portion of the lead illustrated in Figure 1, illustrating the construction of the tip-ring assembly.
Figure 3 is a cross-sectional view of the lead of Figure 1, taken between the coil electrodes mounted thereon.
Figure 4 is a sectional view through the ring-coil spacer component illustrated in Figure 2.
Figure 5 is a plan view of the distal end of the ring-coil spacer component.
Figure 6 is a plan view of the proximal end of the ring-coil spacer component.
Figure 7 is a sectional view through the tip-ring spacer component illustrated in Figure 2.
Figure 8 is a plan view of the proximal end of the tip-ring spacer component.
Figure 9 is a sectional view of the lead of Figure 1 in the vicinity of one of the coil defibrillation electrodes.
Figure 10 is a cutaway view of a portion of the lead of Figure 1 adjacent the connector assemblies.
Figure 11 is a sectional view through a portion of one of the connector assemblies of the lead of Figure 1.

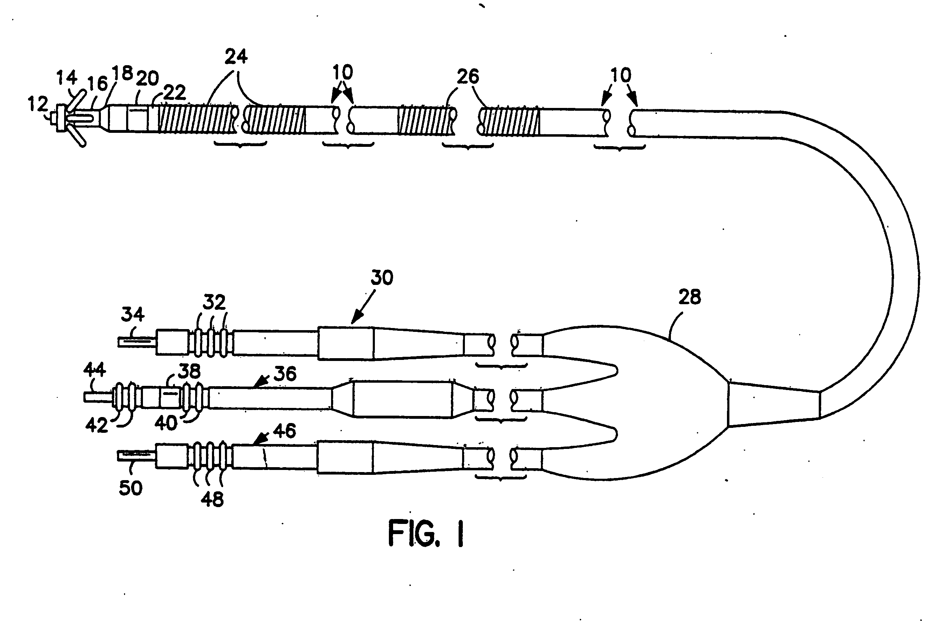

Figure 1 is a plan view of a lead according to the present invention, embodied as a transvenous cardiac defibrillation lead. The lead is provided with an elongated lead body 10 which carries four mutually insulated conductors therein, not visible in this view. Three of the insulated conductors are stranded or cabled conductors, each coupled to one of ring electrode 20, distal coil electrode 24 and proximal coil electrode 26. A fourth, coiled conductor.is coupled to distal or tip electrode 12. The distal portion of the lead includes the tip-ring assembly which includes the tip or distal electrode 12, the tine sheath 16 carrying tines 14, the tip-ring spacer component 18, the ring electrode 20 and the ring-coil spacer component 22. These components together provide a generally rigid assembly, with the tine sleeve 16 fabricated of silicone rubber or relatively softer polyurethanes, and the tip-ring and ring-tip spacers 18 and 22 are fabricated of relatively harder plastics having a shore hardness of at least 75 D, for example polyurethane having a Shore hardness of at least 75D, to provide a relatively rigid assembly extending to the distal end of distal defibrillation electrode 24.

At the proximal end of the lead body are three connector assemblies 30, 36 and 46, extending from a molded trifurcation sleeve 28, typically formed of silicone rubber. Connector assembly 30 carries a single connector pin 34, coupled to the conductor coupled to the distal coil electrode 24, and is provided with sealing rings 32 to seal the connector assembly 30 within the connector bore of an associated implantable cardioverter/defibrillator. Likewise, connector assembly 46 is provided with a single connector pin 50 coupled to the conductor coupled to the proximal coil electrode 26, and is provided with sealing rings 48. Connector assembly 36 takes the form of an IS-1 type connector assembly provided with a connector pin 44 coupled to the coiled conductor extending to tip electrode 12 and a connector ring 38 coupled to a cabled conductor extending to ring electrode 20. Sealing rings 40 and 42 seal the connector assembly within the connector bore of an associated cardioverter/defibrillator and seal between connector pin 44 and connector ring 38. The lead body 10 which extends from the trifurcation sleeve 28 to the tip-ring assembly at the distal end of the lead is preferably formed of an extruded multi-lumen tube, formed of a plastic substantially less rigid than the ring-tip and tip-ring spacer components 18 and 22. Lead body 10 may for example be formed of silicone rubber and/or a relatively softer implantable polyurethane such as those typically employed in transvenous cardiac lead bodies. In the areas between coil electrodes 24 and 26 and in the area between coil electrode 26 and trifurcation sleeve 28, the lead body is provided with an overlay tubing having essentially the same outer diameter as coil electrodes 24 and 26, which may also be fabricated of silicone rubber, polyurethane or the like.

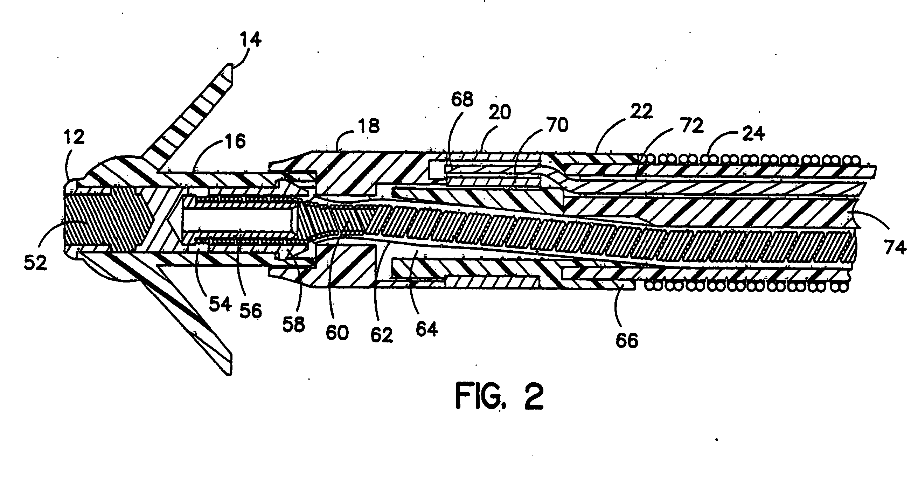

Figure 2 is a sectional view through the tip ring assembly of the lead of Figure 1. At the distal end of the assembly is the distal or tip electrode 12 which is provided with an elongated proximally extending shank around which the tine sleeve 16 is mounted. Electrode 12 may be fabricated of platinum/iridium alloy or other biocompatible metal typically used for cardiac pacing electrodes. The shank portion of electrode 12 contains a proximal facing bore in which a monolithic controlled release device 52 is located, containing an anti-inflammatory steroid such as dexamethasone compounded into a plastic matrix, for example as disclosed in U.S. Patent No. 4,972,848 issued to DiDomenico or 4,506,680 issued to Stokes, or as implemented in any of the various commercially available steroid eluting cardiac pacing leads.

The shank portion of the electrode 12 also contains a distally facing bore in which the distal end of coiled conductor 60 is located. The distal end of coiled conductor 60 is maintained within the shank by means of a crimping or swaging core 56, with conductor 60 compressed between the electrode 12 and the crimping or swaging core 56. Cross bores 54 are provided through the distal portion of the shank of the electrode, allowing for verification of proper placement of coiled conductor 60 during crimping. The distal-most portion of the shank of the electrode 12 includes a radially extending, distally facing flange 58 which engages with a corresponding internally directed proximally facing circumferential flange, molded into tine sleeve 16. Tine sleeve 16 is preferably fabricated of silicone rubber or a relatively softer polyurethane, for example having a Shore hardness of 80A.

Tine sleeve 16 is adhesively bonded to the tip-ring spacer component 18, for example using silicone medical adhesive or a polyurethane based adhesive, depending on the material of tine sleeve 16. Component 18 overlaps the proximal end of the shank of electrode 12 and the proximal end of tine sleeve 16. Component 18 is provided with a proximally facing internal lumen into which the portion 66 of the ring-coil spacer 22 is inserted. The tip-ring spacer and ring-coil spacer 18 and 22 together define a circumferential groove with corresponding proximal and distal facing shoulders which retain ring electrode 20, when assembled. Components 18 and 22 are preferably fabricated of a relatively more rigid plastic than the tine sleeve 16, for example of polyurethane having a Shore hardness of 75D.

A length of PTFE tubing 62 is heat shrunk around coiled conductor 60 and at least the distal portion of the outer surface thereof has been treated to render the tubing bondable, for example by etching by means of the process commercially available from Zeus Industrial Products, Inc., Orangeburg, South Carolina. Alternative surface treatments may also be employed to render the tubing bondable, for example using plasma etching or adhesion promoters as described in U.S. Patent No. 4,944,088 issued to Doan et al. This tubing 62 extends the over the length of the coiled conductor 60 between electrode 12 and connector assembly 36. After assembly, the unfilled space 64 within the tine sleeve 16 and tip-ring and ring-coil spacers 18 and 20 is backfilled with adhesive, bonding the components to themselves and to the etched PTFE tubing 62 and providing for mechanical interlock of all of these components to provide a generally rigid assembly extending from the distal electrode 12 to the distal coil electrode 24.

In this view it can be seen that the ring electrode 20 is provided with an inwardly extending lug 70 having a longitudinal bore into which the distal end of a stranded or cabled conductor 68 has been inserted and which is maintained therein by means of crimps applied to the lug 70. By this mechanism, and in conjunction with the adhesive and mechanical interconnection of the components of the tip-ring assembly shrink tube 62, tensile forces applied to the proximal end of the lead are transmitted to the tip-ring assembly, facilitating removal of the lead without breakage or partial disassembly of the distal portion of the lead.

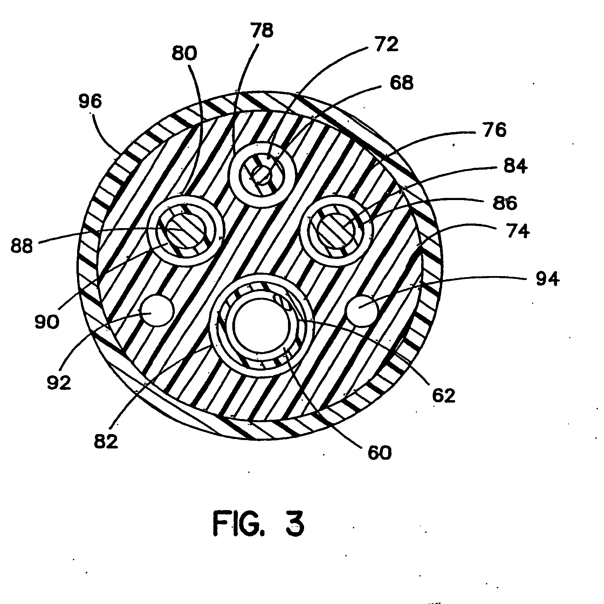

A molded multi-lumen lead body 74 is fabricated of a material softer than the components 18 and 22, for example extruded silicone rubber, or polyurethane having a Shore, for example of 80A or 90A, or the like. Lead body 74 is inserted into a proximal facing recess within ring-coil spacer component 22, and is bonded adhesively therein, for example using a polyurethane or silicone based adhesive. The configuration of the lead body in cross-section is illustrated in more detail in Figure 3. Coiled conductor 60 and cabled conductor 68 each extend proximal to the connector assembly through lumens in extruded lead body 74. Cabled conductor 68 as illustrated is provided with an ETFE coating 72 which is in turn bonded to the interior of the lumen of extruded lead body 74 in which it is located. At least the distal outer surface of insulation 72 is treated to render it bondable, for example using any of the mechanisms discussed above. Alternatively, the coating 72 may be ETFE which has been modified by exposure to gas plasma, for example using an apparatus as described in U.S. Patent No. 5,364,662 issued to DiDomenico et al. with silane used as the feed gas, and ETFE as the plastic to be surface treated.

Coil electrode 24 in this view is visible as having essentially the same outer diameter as the proximal portion of the ring-coil spacer component 22, whereby an essentially isodiametric profile is maintained from the tip-ring spacer up to and including the coil electrode 24. As will be discussed further, this isodiametric profile is maintained proximal to the illustrated portion of the lead by means of overlay tubing, mounted between the electrode coils and between the proximal electrode coil and the trifurcation sleeve (not illustrated in this Figure).

Figure 3 illustrates a cross-section through the body of the lead of Figure 1 in an area intermediate the proximal and distal electrode coils. The lead body 74 is visible in cross-section, and is provided with the total of six lumens extending therethrough, including a first lumen 82 in which the coiled conductor 60, coupled to tip electrode 12 (Fig. 2) is located. The PTFE tubing 62 surrounding coiled conductor 60 is also visible in this view. In second and third lumens 76 and 80 are located stranded conductors 84 and 88, each provided with an insulative coating, 86, 90 of ETFE. Conductors 84 and 88 couple the proximal and distal coil electrodes 24 and 26 (Fig. 1) to their associated connector pins at the proximal end of the lead. A fourth lumen 78 carries stranded or cabled conductor 68 which is coupled to ring electrode 20 (Fig. 2). PTFE coating 72, rendered bondable as discussed above by treatment with silane gas plasma or otherwise, is also visible surrounding stranded conductor 68. Compression lumens 92 and 94 are provided to enhance the ability of the lead to resist crush as described in the above cited Shoberg et al patent, and are located diametrically opposite lumens 80 and 76.

Stranded conductors 84, 88, and 68 may correspond to those described in the Shoberg et al., Williams et al. and/or Laske et al. patents cited above. The number and configuration of the individual strands within the conductor may vary as a function of the expected level of current to be carried by the conductors and as a function of the material of which they are fabricated. Typically, it is expected that in the context of a pacing/cardioversion/defibrillation lead, the conductors be fabricated of MP35N alloy wire or silver cored MP35N wire. Coiled conductor 60 may be a monofilar or multifilar coiled conductor, for example having one through five filars, and corresponds to commonly employed coiled conductors used in implantable pacing leads. The coiled conductor 60 may be also fabricated of MP35N alloy or silver cored MP35N wire.

Surrounding the outer periphery of the lead body 74 is overlay tubing 96, which has approximately the same outer diameter and the same thickness as the wire from which the coil electrodes 24 and 26 are fabricated, providing for an essentially isodiametric assembly extending from the proximal coil electrode 26 to the tip-ring assembly illustrated in Figure 2. A corresponding second overlay tubing extends around lead body 74 proximal to coil electrode 26 (not visible in this view).

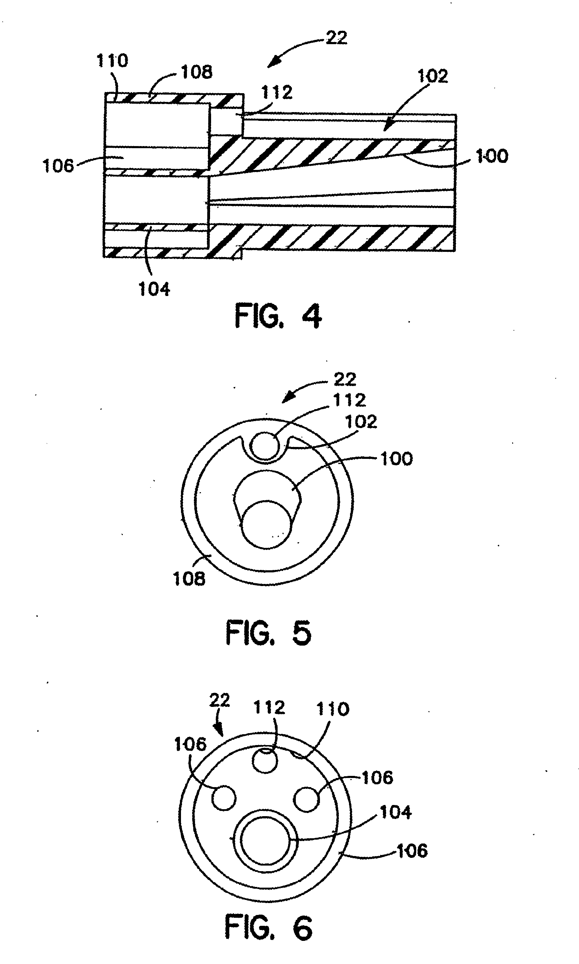

Figure 4 is a cutaway view through the ring-coil spacer 22. The orientation of the component in this figure is reversed from that in Figure 2. The ring-coil spacer component 22 is provided with a through lumen 100, through which the coiled conductor 60 extends (Fig.2). The component is additionally provided with a generaly V-shaped groove 102 in which the lug 70 of the ring electrode 20 (Fig. 2) is located. The proximally facing end of the component 22 is provided with a recess 110 which receives the distal portion of the lead body 74 (Fig. 2). The recess 110 is surrounded by a circumferential wall 108 which has an outer diameter isodiametric to that of the distal coil electrode 24. Extending proximally within recess 110 is a cylindrical sleeve 104 which is inserted into lumen 82 of lead body 74 (Fig. 3). Two proximally extending pins 106 are also located within recess 110 and are configured to be inserted into lumens 80 and 76 of lead body 74 (Fig. 3). Bore 112 allows for passage of the stranded or cabled conductor 68 from the lead body 74 into the lug of the ring electrode 20 (Fig. 2). Figure 5 is a plan view of the distal end of the ring-coil spacer 22, illustrating the relationship of the U-shaped groove 102, the bore 112, the through lumen 100 and the circumferential wall 108 in more detail. Figure 6 is a plan view of the proximal end of the component 22, illustrating the relative locations of the circumferential wall 106, the cylindrical sleeve 104, pins 106 and bore 108, in more detail.

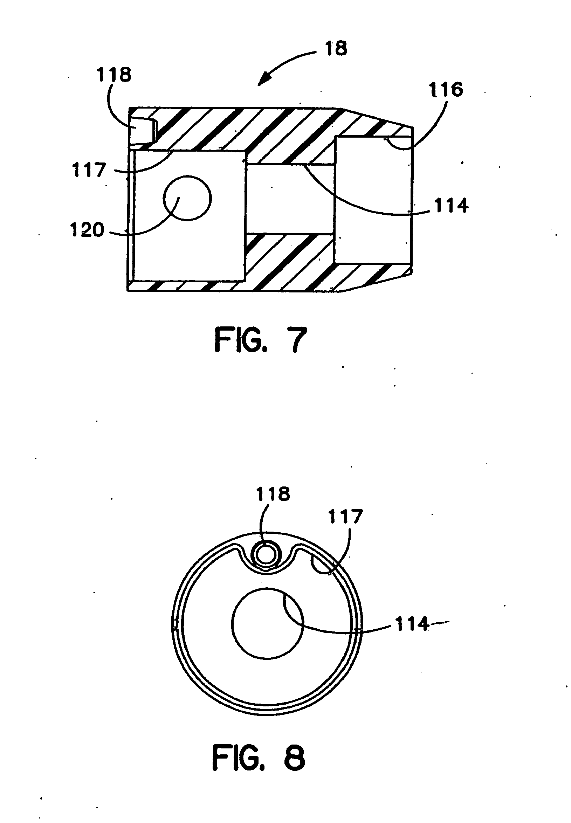

Figure 7 is a sectional view through the tip-ring spacer 18. Again, the orientation of this view is reversed from that illustrated in Figure 2. The tip-ring spacer 18 is provided with a through lumen 114, through which the coiled conductor 60 (Fig. 2) extends. A distal-facing recess 116 receives the proximal end of the tine sleeve 16 (Fig. 2) and overlaps the proximal portion of the shank of electrode 12 (Fig. 2). A proximal facing recess 117 receives the distal portion of component 22 as illustrated in Figures 4-6. A small proximally facing lumen 118 is provided, which as assembled is aligned with the bore through the lug 70 of ring electrode 20 (Fig. 2), providing a recess into which the cabled or stranded conductor 68 may extend. Bores 120 are provided through the sidewall of component 18, allowing for backfilling of the recess 64 internal to the tip-ring assembly, as illustrated in Figure 2. Figure 8 is a plan view of the proximal end of component 18 and illustrates the configuration of the recess 117 which receives the distal portion of component 22, through lumen 114 and recess 118 in more detail.

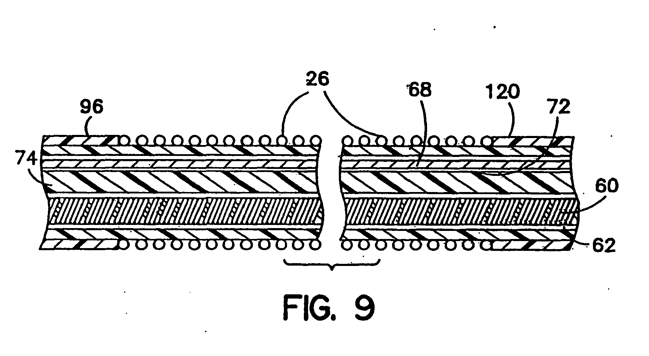

Figure 9 is a sectional view through the lead of Figure 1 in the vicinity of the proximal coil electrode 26. Coil electrode 26 is shown located around lead body 74, flanked on its proximal and distal ends by overlay tubing 96 and 120. Overlay tubing 96 corresponds to the same element illustrated in Figure 3 and extends between coil electrodes 24 and 26. Overlay tubing 120 extends to the trifurcation sleeve 28, illustrated in Figure 1. Together the coil electrodes 24 and 26 in conjunction with the overlay tubing 96 and 120 provide an essentially isodiametric lead body extending from the trifurcation sleeve to the tip-ring assembly illustrated in Figure 2. Also visible in this view are stranded or cabled conductor 68 and associated insulative coating 72 and coiled conductor 60 and associated heat shrink PTFE tubing 62. Although not illustrated in Figure 9 it should be understood that the coil electrodes 24 and 26 may be coupled to stranded or cabled conductors 88 and 84 (Fig. 3) by means of cross-groove crimp sleeves of the sort described in the above cited patent issued to Boser et al.

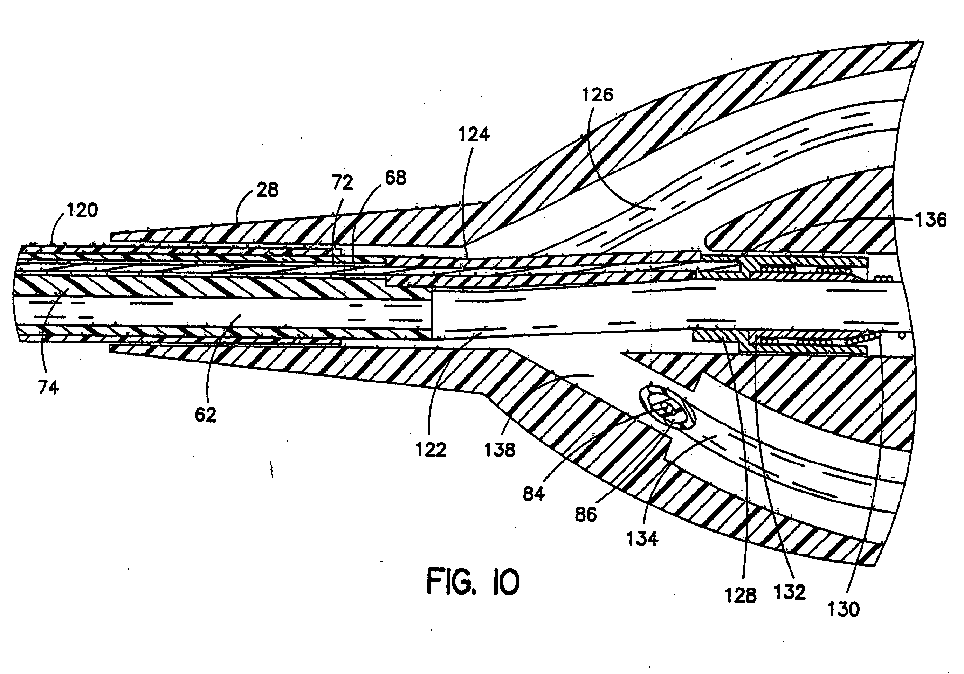

Figure 10 illustrates a cutaway view through the lead of Fig. 1 in the vicinity of trifurcation sleeve 28. Lead body 74 enters the distal end of trifurcation sleeve 28 and terminates therein. Stranded or cabled conductor 68 extends through lead body 74, out its proximal end and through spacer tubing 124 which extends to transition flange 128, which in turn contains a bore 136 in which the proximal end of cabled or stranded conductor 68 is crimped. At least the proximal outer surface ofETFE insulative coating 72 applied to conductor 68 is made bondable using one of the methods discussed above and as adhesively bonded to the lumen of lead body 74, in the area adjacent to the point at which it exits lead body 74. This adhesive bond provides for a mechanical interconnection between the conductor 68 and the lead body 74, in region of the trifurcation sleeve, which in turn enhances the ability to transmit tensile force provided by the mechanical and electrical interconnection of the stranded or cabled conductor 68 to transition sleeve 128. A coiled conductor 130 is coupled to transition sleeve 128 by means of a crimping or swaging core 132. Connector 130 extends proximally to the IS-1 connector assembly 36 (Fig. 1) where it is coupled to connector ring 38 in a conventional fashion.

Also visible in this view is PTFE shrink tubing 62 which surrounds the coiled conductor 60 (Fig. 2). Shrink tubing 62 and conductor 60 extend proximally inside inner tubing 122 which also extends proximally to the IS-1 connector assembly 36. As discussed below, PTFE shrink tubing 62 is adhesively bonded to the interior of inner tubing 122, in the vicinity of IS-1 connector 36, further enhancing the ability of the lad to transmit tensile force from the proximal to the distal tip of the lead. Also visible in this view are two insulative tubes 126 and 134, each of which surrounds one of the stranded conductors coupled to a coil electrode, and which extend back to the connector assemblies 30 and 46, illustrated in Figure 1. Tube 134, for example, carries conductor 84 and associated insulative coating 86. The recess 138 defined within trifurcation sleeve 28 is backfilled with silicone rubber medical adhesive, providing a mechanical interconnection of all the components therein. This mechanical interconnection also assists in mechanically coupling the proximal end of the lead body to IS-1 connector assembly 36 and trifurcation sleeve 28.

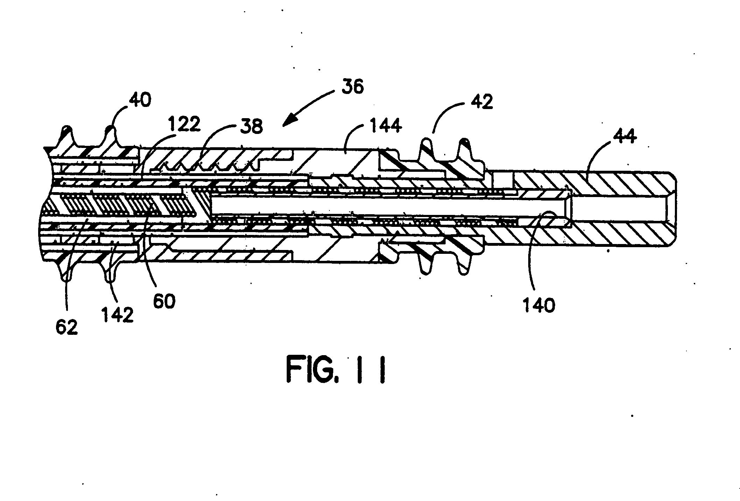

Figure 11 is a cutaway view through IS-1 connector assembly 36, illustrating the interconnection of the various components including the connector ring 38, connector pin 44 and sealing rings 40 and 42. As illustrated, coiled conductor 60 is coupled to connector pin 44 by means of a crimping or swaging core 140. Coiled conductor 60 and its associated PTFE shrink tubing 62 are located within inner tubing 122, which extends proximally from trifurcation sleeve 28, as illustrated in Figure 10. Ring electrode 38 is provided with cross bores 142 which facilitate backfilling of the recess between the ring electrode 38 and the inner tubing 122, serving to mechanically interconnect the inner tubing 122 to ring electrode 38 and sealing rings 40. Ring electrode 38 is in turn mechanically interconnected with connector pin 44 by means of injection molded spacer 144, fabricated according to U.S. Patent No. 4,572,605 issued to Hess. At least the proximal outer surface of PTFE shrink tubing 62 applied to conductor 60 is made bondable using one of the methods discussed above and is adhesively bonded to the lumen of inner tubing 122, further facilitating transmission of tensile forces from the proximal end to the distal end of the lead body, as discussed above.

The above disclosed embodiment of a lead according to the present invention takes the form of a cardioversion/defibrillation lead which is provided with four electrodes including a tip electrode, a ring electrode and two defibrillation electrodes and which employs all three of the described extraction enhancing features in combination. Variations of the invention, using one or more of the features enumerated herein particularly adapted to assist in rendering the lead extractable, may of course be used in conjunction with leads having a greater or fewer number of electrodes and conductors.

## Claims

1. A medical electrical lead comprising:
an elongated lead body (74) having proximal and distal ends and comprising plastic lead components;
an electrical connector (30, 36, 46) mounted to a proximal portion of the lead body; further comprising
a tip-ring assembly comprising
three of said plastic components (14, 18, 22);
a first, tip electrode (12) mounted to a distal portion of the lead body and a ring electrode (20) located proximally to the tip electrode (12) the three plastic components of the tip-ring assembly being molded plastic components and being configured to provide mechanical interlock between the tip electrode and the ring electrode when assembled, and two of the three plastic components of the tip-ring assembly being fabricated of a plastic having a Shore hardness of at least 75D, said lead further comprising
an elongated coiled conductor (60) mounted in said lead body and coupled to said first, tip, electrode, provided with a covering (62) of an insulative flouropolymer extending between a first point along the proximal portion of the lead body (74) and a second point along the distal portion of the lead body (74), treated to be adhesively bondable at least adjacent the first and second points and adhesively bonded to said plastic components of said tip-ring assembly at said second point and to a plastic component of the lead at said first point.

2. A lead according to claim 1 wherein the lead body (10) comprises an elongated plastic tube (74) and wherein the covering (62) is bonded to the plastic tube at said first point.

3. A lead according to claim 1 or claim 2 wherein the lead body comprises molded plastic parts located at the first point and wherein the covering (62) is bonded to at least one of the molded plastic parts.

## Patentansprüche

1. Medizinische elektrische Leitung, mit:
einem lang gestreckten Leitungskörper (74), der ein proximales und ein distales Ende besitzt und Kunststoffleitungskomponenten aufweist;
einem elektrischen Verbinder (30, 36, 46), der an einem proximalen Abschnitt des Leitungskörpers angebracht ist; ferner mit
einer Spitzenringanordnung, die enthält:
drei der Kunststoffkomponenten (14, 18, 22);
eine erste Spitzenelektrode (12), die an einem distalen Abschnitt des Leitungskörpers angebracht ist, und eine Ringelektrode (20), die sich in der Nähe der Spitzenelektrode (12) befindet, wobei die drei Kunststoffkomponenten der Spitzenringanordnung gegossene Kunststoffkomponenten sind und konfiguriert sind, um eine mechanische Verriegelung zwischen der Spitzenelektrode und der Ringelektrode zu schaffen, wenn sie zusammengefügt sind, und wobei zwei der drei Kunststoffkomponenten der Spitzenringanordnung aus Kunststoff mit einer Shore-Härte von wenigstens 75 D hergestellt sind, wobei die Leitung ferner enthält:
einen lang gestreckten gewundenen Leiter (60), der in dem Leitungskörper angebracht ist und mit der ersten Spitzenelektrode gekoppelt ist und mit einer Abdeckung (62) aus einem isolierenden Fluorpolymer versehen ist, die sich zwischen einem festen Punkt längs des proximalen Abschnitts des Leitungskörpers (74) und einem zweiten Punkt längs des distalen Abschnitts des Leitungskörpers (74) erstreckt und so behandelt ist, dass sie wenigstens benachbart zu dem ersten und dem zweiten Punkt klebend haften können und an den Kunststoffkomponenten der Spitzenringanordnung an dem zweiten Punkt und an einer Kunststoffkomponente der Leitung an dem ersten Punkt klebend haftet.

2. Leitung nach Anspruch 1, wobei der Leitungskörper (10) ein lang gestrecktes Kunststoffrohr (74) aufweist und wobei die Abdeckung (62) an dem Kunststoffrohr an dem ersten Punkt haftet.

3. Leitung nach Anspruch 1 oder Anspruch 2, wobei der Leitungskörper gegossene Kunststoffteile aufweist, die sich an dem ersten Punkt befinden, und wobei die Abdeckung (62) an wenigstens einem der gegossenen Kunststoffteile haftet.

## Revendications

1. Fil électrique médical comportant :
un corps de fil allongé (74) ayant des extrémités proximale et distale et comportant des composants de fil en matière plastique ;
un connecteur électrique (30, 36, 46) monté sur une partie proximale du corps de fil ; comportant en outre un ensemble pointe - anneau comportant
trois desdits composants en matière plastique (14, 18, 22) ;
une première électrode en pointe (12) montée sur une partie distale du corps de fil et une électrode en anneau (20) localisée à proximité de l'électrode en pointe (12), les trois composants en matière plastique de l'ensemble pointe - anneau étant des composants en matière plastique moulés et étant configurés pour assurer un verrouillage mécanique entre l'électrode en pointe et l'électrode en anneau lorsqu'assemblées, et deux des trois composants en matière plastiques de l'ensemble pointe - anneau étant constitués d'une matière plastique ayant une dureté Shore d'au moins 75D, ledit fil comportant en outre
un conducteur spiralé allongé (60) monté dans ledit corps de fil et couplé à ladite première électrode en pointe, muni d'un revêtement (62) constitué d'un fluoropolymère isolant s'étendant entre un premier point le long de la partie proximale du corps de fil (74) et un second point le long de la partie distale du corps de fil (74), traité pour pouvoir être fixé de manière adhésive et au moins adjacent aux premier et second points et fixé de manière adhésive aux-dits composants en matière plastique dudit ensemble pointe - anneau au niveau dudit second point et à un composant en matière plastique du fil au niveau dudit premier point.

2. Fil selon la revendication 1, dans lequel le corps de fil (10) comporte un tube en matière plastique allongé (74) et dans lequel le revêtement (62) est fixé au tube en matière plastique au niveau dudit premier point.

3. Fil selon la revendication 1 ou 2, dans lequel le corps de fil comporte des parties en matière plastique moulées positionnées au niveau du premier point et dans lequel le revêtement (62) est fixé à au moins l'une des parties en matière plastique moulées.
